# EUROPEAN PATENT APPLICATION

(11) **EP 1 712 214 A1**
(43) Date of publication of application: **18.10.2006**
(21) Application number: 04707293.9
(22) Date of filing: 02.02.2004
(51) Int. Cl.: A61F 13/53

(54) **ABSORBENT ARTICLE**

(71) Applicant: JAPAN ABSORBENT TECHNOLOGY INSTITUTE, Chuo-ku, Tokyo 103-0007 (JP)
(72) Inventor: SUZUKI,Migaku; c/o Japan Absorbent Tec. Inst., Chuo-ku, Tokyo 103-0007 (JP); ANDO, Katsumi; c/o Japan Absorbent Tec. Inst., Chuo-ku, Tokyo 103-0007 (JP); TAKEMATSU, Satomi, Chiyoda-ku, Tokyo 100-8086 (JP); SEKIGUCHI,Hiroko; c/o Japan Absorbent Tec. Inst., Chuo-ku, Tokyo 103-0007 (JP); MORIYA, Reiko; c/o Japan Absorbent Tec. Inst., Chuo-ku, Tokyo 103-0007 (JP)
(86) International application number: PCT/JP2004/000991
(87) International publication number: WO 2005/072672

(57) **Abstract**

It is an object of the present invention to provide an absorbent article which does not cause discomfort to a wearer when worn, particularly after an absorber absorbs a body fluid to be swelled, and which is capable of receiving urine and feces separately. The object is attained by an absorbent article, including: a leak preventer having a sheet, and two bags provided separately on right and left of an upper side of the sheet, each of the two bags having an opening facing with each other; and an absorber having at least one layer and containing a super absorbent polymer and capable of absorbing a body fluid, provided in each of the two bags.

## Description

### Technical Field

The present invention relates to a novel absorbent article.

### Background Art

An absorbent article such as a disposable diaper, a sanitary napkin, or an incontinence article is an article for absorbing a body fluid such as urine excreted from a wearer into an absorber employing a super absorbent polymer (hereinafter, may also be referred to as "SAP"). A conventional absorbent article has a structure for absorbing a body fluid mainly at the crotch of the body.

However, the crotch is narrow, and the conventional absorbent article has a problem of causing discomfort to a wearer when worn. This discomfort is significant after the absorber absorbs a body fluid to be swelled.

Further, the conventional absorbent article has a structure for covering a urethral meatus (external urethral meatus) and an anus, and thus, disposing urine and feces separately is difficult. When the urine and feces mix together while the absorbent article is used, a problem of causing a rash, an offensive odor, or the like develops.

### Disclosure of the Invention

It is an object of the present invention therefore to provide an absorbent article which does not cause discomfort to a wearer when worn, particularly after an absorber absorbs a body fluid to be swelled, and which is capable of disposing urine and feces separately.

The inventors of the present invention have conducted intensive studies to attain the above-mentioned object, and have completed an absorbent article having a novel structure.

That is, the present invention provides the following items (1) to (33).
(1) An absorbent article, including:
   a leak preventer having a sheet, and two bags provided separately on right and left of an upper side of the sheet, each of the two bags having an opening facing with each other; and
   an absorber having at least one layer and containing a super absorbent polymer and capable of absorbing a body fluid, provided in each of the two bags.
(2) The absorbent article according to the above item (1), in which the two bags are symmetrical.
(3) The absorbent article according to the above item (1) or (2), further including a connecting absorber that connects the absorbers provided in each of the two bags.
(4) The absorbent article according to any one of the above items (1) to (3), in which the absorbers are sheet absorbers.
(5) The absorbent article according to the above item (4), in which the sheet absorbers contain 50 wt% or more of the super absorbent polymer.
(6) The absorbent article according to the above item (4) or (5), in which the sheet absorbers having multiple layers are provided in the bags.
(7) The absorbent article according to any one of the above items (4) to (6), in which the sheet absorbers are provided in the bags while being folded.
(8) The absorbent article according to any one of the above items (1) to (7), in which an area of the sheet where the two bags are not provided occupies 50% or less of an area of the entirety of the sheet in the leak preventer.
(9) The absorbent article according to any one of the above items (1) to (8), in which a ratio of an average left-to-right length W to an average front-to-rear length L is 0.5 or more.
(10) The absorbent article according to any one of the above items (1) to (9), in which an average front-to-rear length L is 20 cm or less and an area of the leak preventer seen from an upper side thereof is 400 cm² or less.
(11) The absorbent article according to any one of the above items (1) to (10), further including a guide sheet bridging the absorbers provided in each of the two bags.
(12) The absorbent article according to the above item (11), in which the guide sheet allows transfer of a body fluid between the absorbers provided in each of the two bags.
(13) The absorbent article according to the above item (11) or (12), in which the guide sheet extends to cover a part or entire lower surface of each of the absorbers.
(14) The absorbent article according to the above item (13), in which the guide sheet extends to further cover a part or entire side surface of each of the absorbers.
(15) The absorbent article according to any one of the above items (11) to (14), in which the guide sheet includes a concavity-and-convexity-containing sheet member having apertures forming flow paths.
(16) The absorbent article according to the above item (15), in which the guide sheet further includes a hydrophilic diffusion sheet laminated under or combined to a lower surface of the concavity-and-convexity-containing sheet member.
(17) The absorbent article according to the above item (16), in which the guide sheet further includes a body fluid impermeable sheet laminated under or combined to a lower surface of the hydrophilic diffusion sheet.
(18) The absorbent article according to any one of the above items (1) to (17), further including a skin contact sheet at least between the two bags of the leak preventer.
(19) The absorbent article according to any one of the above items (1) to (18), further including a spacer between an inner surface of an upper side of each of the bags of the leak preventer and an upper surface of each of the absorbers.
(20) The absorbent article according to any one of the above items (1) to (19), further including a liquid leak prevention barrier on an upper surface of the opening of each of the bags.
(21) The absorbent article according to any one of the above items (1) to (20), further including a fitting member for fitting the absorbent article to a body surface of a wearer between the two bags of the leak preventer.
(22) The absorbent article according to the above item (21), in which the fitting member is provided on an upper side of the sheet of the leak preventer.
(23) The absorbent article according to the above item (21) or (22), in which the fitting member is provided under a lower side of the sheet of the leak preventer.
(24) The absorbent article according to any one of the above items (21) to (23), in which a front-to-rear length of the fitting member is substantially the same as a front-to-rear length of the leak preventer.
(25) The absorbent article according to any one of the above items (21) to (23), in which a front-to-rear length of the fitting member is longer than a front-to-rear length of the leak preventer.
(26) The absorbent article according to any one of the above items (21) to (25), in which the fitting member is bonded to the leak preventer.
(27) The absorbent article according to any one of the above items (21) to (26), including the fitting member having elasticity.
(28) The absorbent article according to any one of the above items (21) to (27), including the fitting member having stretchability.
(29) The absorbent article according to the above item (28), including a plurality of the fitting members.
(30) The absorbent article according to the above item (28) or (29), in which the fitting member is branched on a front side and/or a rear side.
(31) The absorbent article according to any one of the above items (21) to (30), further including a feces-receiving portion.
(32) An absorbent article set including: the absorbent article according to any one of the above items (1) to (31); and a cover.
(33) The absorbent article set according to the above item (32), in which the cover includes a fitting member for fitting the absorbent article to a body surface of a wearer between the two bags of the leak preventer.

### Brief Description of Drawings

FIGs. 1 are schematic diagrams each showing an example of an absorbent article of the present invention.
FIGs. 2 are schematic diagrams each explaining a usage of an absorbent article of the present invention.
FIGs. 3 are schematic cross sectional diagrams showing two examples of a leak preventer having a composite sheet.
FIGs. 4 are schematic diagrams showing two examples of a leak preventer having pleats with each of bags.
FIGS. 5 are schematic diagrams each showing a leak preventer and an absorber having a space between an inner surface of a bag of the leak preventer and an upper surface of the absorber.
FIGs. 6 are schematic cross sectional diagrams showing other examples of an absorbent article of the present invention.
FIG. 7 is a schematic perspective view showing an example of a sheet member having a grooved structure.
FIG. 8 is a schematic perspective view showing an example of a concavity-and-convexity-containing sheet member.
FIG. 9 is a schematic perspective view showing an example of a concavity-and-convexity-containing sheet member having an opening in each of convex portions.
FIGs. 10 are schematic cross sectional diagrams explaining various arrangements of a guide sheet.
FIGs. 11 are schematic partial cross sectional diagrams showing various structures of a body fluid receiving region of an absorbent article of the present invention.
FIG. 12 is a schematic plan view showing another example of an absorbent article of the present invention.
FIGs. 13 are schematic plan views showing various examples of a leak preventer of an absorbent article of the present invention.
FIGs. 14 are schematic plan views showing various examples of a leak preventer of an absorbent article of the present invention.
FIGs. 15 are schematic cross sectional diagrams showing various examples of an absorbent article of the present invention.
FIGs. 16 are schematic plan views showing examples of an absorbent article of the present invention having a detachable member for a fitting member.
FIGs. 17 are partial plan views schematically showing various examples of an absorbent article set of the present invention consisting of the absorbent article of the present invention and a cover.
FIG. 18 is a longitudinal sectional diagram of the absorbent article set shown in FIG. 17(A) taken along the face passing the crotch of the body when worn.
FIGs. 19 are schematic plan views showing various examples of an absorbent article of the present invention having a feces-receiving portion.
FIGs. 20 are schematic diagrams each showing an absorbent article of the present invention used for a body fluid absorption test.
FIGs. 21 are schematic diagrams each showing an absorbent article of the present invention used for a body fluid absorption test.
FIGs. 22 are schematic diagrams each showing a skin contact sheet, a guide sheet, and a fitting member of an absorbent article of the present invention used for a body fluid absorption test.

### Best Mode for Carrying Out the Invention

Hereinafter, an absorbent article of the present invention will be described in more detail based on preferable embodiments shown in attached drawings. In the specification of the present invention, when the absorbent article is actually worn, a side close to a wearer's skin is referred to as "upper" side and a side far therefrom is referred to as "lower" side. In addition, when the absorbent article is actually worn, a side corresponding to a front side of a body of a wearer is referred to as "front" and a side corresponding to a rear side thereof is referred to as "rear". Note that, in the drawings, members which are actually in contact with each other may be shown to be separately disposed for easy understanding.

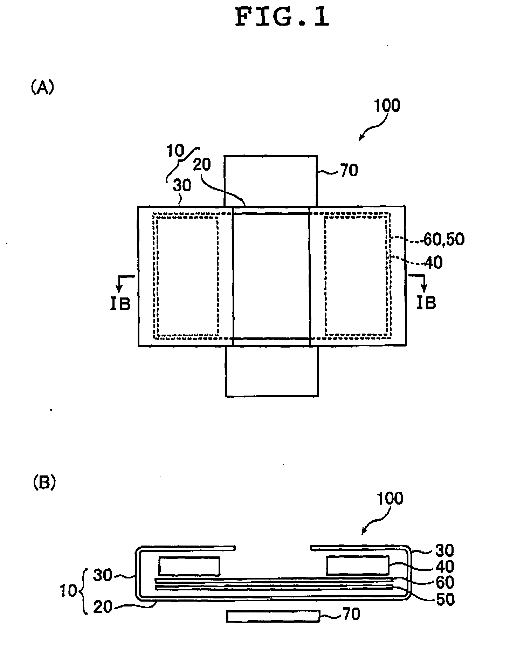

FIGs. 1 are schematic diagrams each showing an example of an absorbent article of the present invention. FIG. 1(A) is a plan view and FIG. 1(8) is a cross sectional diagram taken along the line IB-IB of FIG. 1(A). Note that, in each plan view, a front side of an absorbent article and others is shown on an upper side of the diagram. In each plan view, when a front side and a rear side are not symmetrical, the front side and the rear side may be reversed.

An absorbent article 100 of the present invention is basically includes: a leak preventer 10 having a sheet 20, and two bags 30 provided separately on right and left of an upper side of the sheet 20, each of the two bags having an opening facing with each other; and absorbers 40 having at least one layer and containing a super absorbent polymer and capable of absorbing a body fluid, provided in each of the two bags 30.

As described above, the leak preventer 10 includes the sheet 20 and the two bags 30. Materials generally used for a back sheet can be used as materials for the sheet 20 and the two bags 30. Specific examples of the materials that can be used include: a resin film of PE, PP, PET, EVA, or the like; and a foamed sheet of the resin. The resin film may be used as a composite sheet of the film and a nonwoven fabric for the better touch or appearance. In this case, an SB nonwoven fabric, an SMS nonwoven fabric, a thermal bond nonwoven fabric, or the like having a relatively light weight can be suitably used as the nonwoven fabric.

The sheet 20 and the two bags 30 may be constructed integrally or may be bonded. The sheet 20 and the two bags 30 each may be constructed of a plurality of members.

A shape of the sheet 20 is not particularly limited, but a preferable aspect thereof is substantially rectangular.

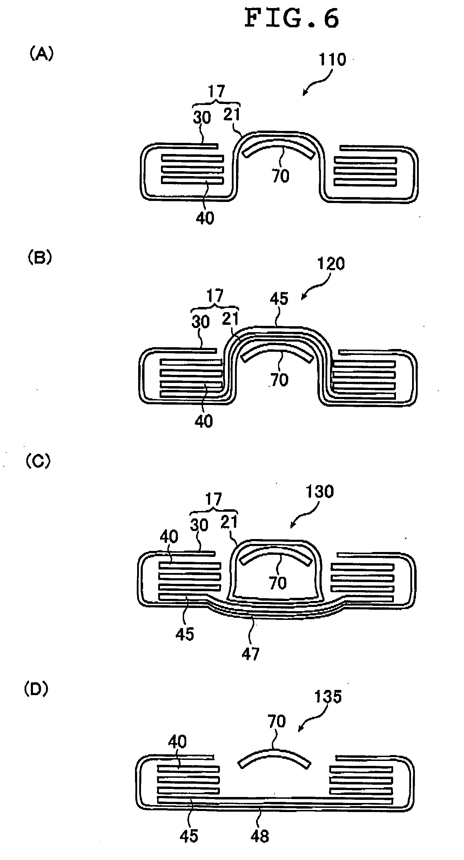

The sheet 20 may be planar as shown in FIGs. 1 or may be folded as shown in FIGs. 6(A) and 6(B).

The two bags 30 are provided separately on right and left of an upper side of the sheet 20 so that apertures thereof face each other. The two bags 30 are preferably symmetrical.

The absorber 40 used in the present invention is not particularly limited as long as it absorbs a body fluid. For example, a powdery absorber may be used, but a sheet absorber is preferable in consideration of form stability, releasing of powder, or the like. As described below, when the absorbent article of the present invention has a guide sheet, form stability becomes important.

The sheet absorber is preferably a super absorbent sheet containing SAP in 50 wt% or more, preferably 60 to 95 wt%.

The super absorbent sheet is an ultrathin sheet absorber containing SAP as a main component. The super absorbent sheet has a very high SAP content, and thus is very thin. The super absorbent sheet has a thickness of preferably 1.5 mm or less, more preferably 1 mm or less.

A structure or production method of the super absorbent sheet is not particularly limited as long as it is an ultrathin sheet absorber containing SAP as a main component.

An example of the super absorbent sheet includes a super absorbent sheet obtained through an Air Laid process. The Air Laid process involves mixing pulverized wood pulp and SAP, adding a binder, and forming the mixture into a sheet, to thereby obtain a super absorbent sheet. Examples of the super absorbent sheet obtained through the process include: NOVATHIN available from Reyonier Inc. (U.S.A.); and B-SAP available from OJI KINOCLOTH CO., LTD.

Another example thereof includes a super absorbent sheet obtained through a process involving coating SAP-dispersed slurry on a body fluid permeable sheet such as a nonwoven fabric. The SAP-dispersed slurry is preferably prepared by dispersing SAP and microfibrillated cellulose (MFC) in a mixed solvent of water and ethanol. An example of the super absorbent sheet obtained through this process includes MegaThin available from Japan Absorbent Technology Institute.

other examples thereof include: a super absorbent sheet obtained through a process involving carrying a large amount of SAP on a raised-state nonwoven fabric and fixing the SAP with a hot melt binder, an emulsion binder, an aqueous fiber, or the like; and a super absorbent sheet obtained through a process involving mixing fiber-form SAP with a PET (polyethylene terephthalate) fiber and forming the mixture into a web.

The super absorbent sheet of at least one layer is provided in each of the two bags 30. That is, the super absorbent sheet provided in each of the bags 30 may be one layer or two or more layers.

The super absorbent sheet may also be provided in each of the bags 30 while being folded.

As shown in FIGs. 1, the absorbent article 100 of the present invention is preferably further provided with a guide sheet 50 bringing the absorbers 40 provided in each of the two bags 30.

The guide sheet 50 has a flow path allowing transfer of a body fluid, and quickly transfers urine discharged from a urethral meatus m of a wearer M to a portion between the two bags 30 of the leak preventer 10 (hereinafter, the portion is referred to as "body fluid receiving region") to the absorbers 40. Thus, the wearer does not feel discomfort at the crotch of the body.

The guide sheet 50 preferably allows transfer of a body fluid between the absorbers 40 provided in the respective two bags 30.

When an absorbent article is used, volumes of a body fluid absorbed in absorbers provided in the respective two bags may be significantly unbalanced depending on a posture of a wearer, a wear position, or the like. In this case, if the absorbent article of the present invention has a guide sheet, a body fluid transfers from an absorber absorbing a large volume of the body fluid to an absorber absorbing a small volume thereof. As a result, the unbalance is alleviated. Thus, an actual absorption volume of a body fluid in the absorbent article and a usable life time of the absorbent article are increased.

The guide sheet 50 is not particularly limited as long as the sheet has a structure including a flow path allowing transfer of a body fluid. The guide sheet 50 preferably has neither body fluid absorbency nor body fluid retentivity for allowing quick transfer of the body fluid.

When an absorbent article is used, the absorbent article is sandwiched between the wearer and underwear or clothes and a pressure is applied thereto. Thus, the guide sheet 50 preferably has pressure resistance. To be specific, a thickness of the absorbent article maintained under a pressure of 10 gf/cm² (9.81 × 10² Pa) is preferably 0.3 mm or more, more preferably 0.5 mm or more, and furthermore preferably 1.0 mm or more. The guide sheet 50 consisting of two or more laminated layers may be used to satisfy the pressure resistance.

A preferable guide sheet will be described later.

As shown in FIGs. 1, the absorbent article 100 of the present invention is preferably further provided with a skin contact sheet 60. Being provided with the skin contact sheet 60 results in excellent surface touch.

A conventionally known sheet can be used for the skin contact sheet. Specific examples thereof that may be used include: a nonwoven fabric of a synthetic fiber such as a pp nonwoven fabric, a PET nonwoven fabric, a PE nonwoven fabric, or a polyolefin-polyester nonwoven fabric; and a fabric obtained by blending synthetic fiber and cotton.

As shown in FIGs. 1, the absorbent article 100 of the present invention is preferably further provided with a fitting member 70.

When the wearer is a female, urine easily leaks to a portion other than the body fluid receiving region because of reasons such as a slightly protruded urethral meatus and a short perineum. In the present invention, the fitting member can fit the body fluid receiving region of the absorbent article to a body surface region including the urethral meatus. Thus, urine quickly transfers to the bags 30 and is absorbed in the absorbers 40. The details of the fitting member will be described later.

Next, a usage of the absorbent article 100 will be described.

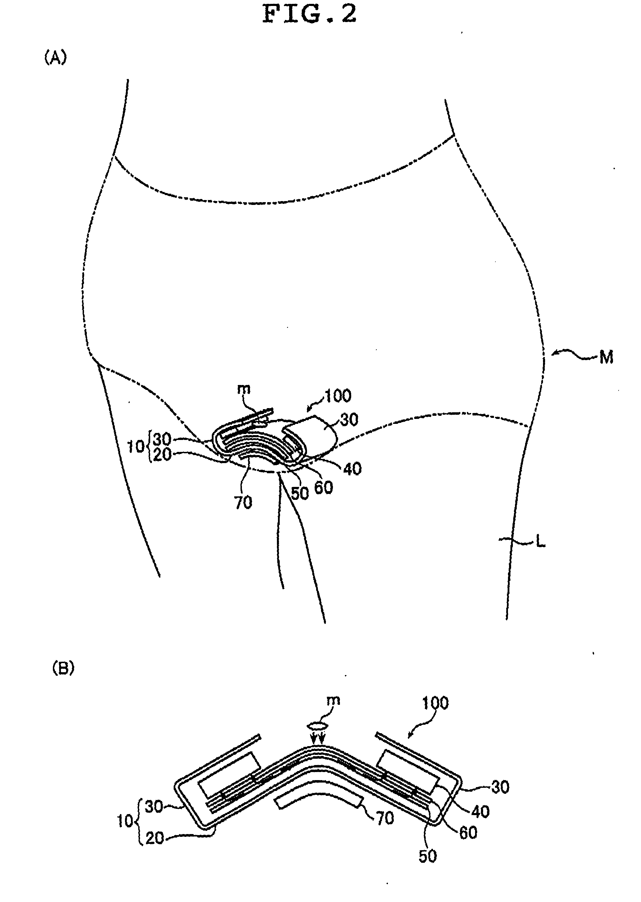

FIGs. 2 are schematic diagrams each explaining a usage of the absorbent article of the present invention. FIG. 2(A) is a partially cutaway cross sectional perspective view showing the absorbent article when worn, and FIG. 2(B) is a cross sectional diagram of the absorbent article.

As shown in FIGs. 2, the absorbent article 100 is worn so that the urethral meatus m of the wearer M is positioned between the two bags 30 of the leak preventer 10 (body fluid receiving region), and that the bags 30 closely contact with inner groin of both legs L of the wearer M. As shown in FIGs. 2, the absorbent article 100 is provided with the fitting member 70, and thus, the fitting member 70 fits the body fluid receiving region of the absorbent article 100 to the body surface region including the urethral meatus m.

Then, the urine discharged from the urethral meatus m of the wearer M passes through the skin contact sheet 60 from the body fluid receiving region by gravity as shown by arrows in FIG. 2(B). The urine flows along the guide sheet 50 to the bags 30 and is absorbed in the absorbers 40 in the bags 30. When the urine is supplied at a rate exceeding an absorption rate of the absorbers 40, the urine not absorbed in the absorbers 40 is accumulated in the bags 30 once, and then is absorbed in the absorbers 40 later.

As described above, the absorbent article of the present invention, when worn, has the absorbers mainly positioned in portions other than the crotch, and thus, causes no discomfort which was significant with the conventional absorbent article particularly after the absorber absorbs the body fluid to be swelled.

The absorbent article of the present invention only needs to have a structure covering a periphery of the urethral meatus. Thus, urine and feces may be received separately.

In particular, when the absorbent article of the present invention is provided with the fitting member, the urine assuredly flows to the bags 30 and is absorbed in the absorbers 40.

Hereinafter, other preferable embodiments of the present invention will be described in detail.

### <Leak preventer and others>

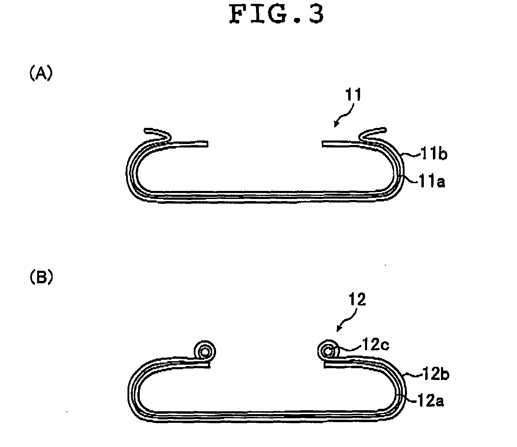

FIGs. 3 are schematic cross sectional diagrams showing two examples of a leak preventer composed of a composite sheet. FIG. 3(A) shows a first example, and FIG. 3(B) shows a second example.

When the leak preventer is a composite sheet of a film and a nonwoven fabric, the film and the nonwoven fabric may be partially not in contact with each other, or the nonwoven fabric may have an elastic member.

In the first example as shown in FIG. 3(A), a composite sheet of a leak preventer 11 is constructed of a film 11a and a nonwoven fabric 11b. In a vicinity of an opening edge portion of each of the bags, the nonwoven fabric 11b is folded back from the film 11a to rise. Such rising portion of the nonwoven fabric prevents a leak of the body fluid outward from the rising portion of the nonwoven fabric even when the body fluid leaks on the film of the opening edge portion of each of the bags from the body fluid receiving region.

In the second example as shown in FIG. 3(B), a composite sheet of a leak preventer 12 is constructed of a film 12a and a nonwoven fabric 12b. In the vicinity of an opening edge portion of each of the bags, the nonwoven fabric 12b is folded back from the film 11a to rise and is rolled to wrap a flexible elastic member (elastic filament, for example) 12c, to serve as a liquid leak prevention barrier. Thus, when the leak preventer has a liquid leak prevention barrier on an upper surface of the opening of each of the bags, the discharged body fluid is hardly leaked to a portion other than the body fluid receiving region.

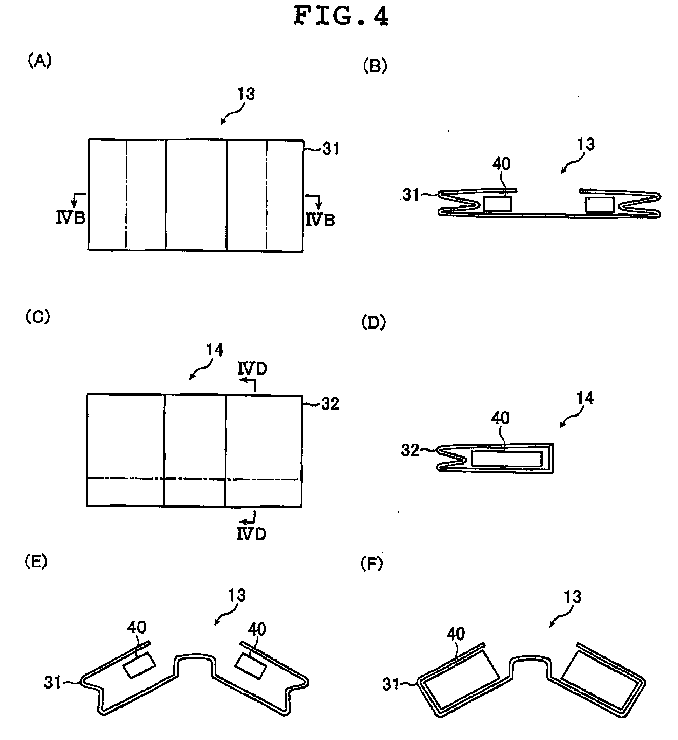

FIGs. 4 are schematic diagrams showing two examples of a leak preventer having pleats with bags. FIG. 4(A) is a plan view showing a first example, and FIG. 4(B) is a cross sectional diagram of the first example taken along the line IVB-IVB of FIG. 4(A). FIG. 4(C) is a plan view showing a second example, and FIG. 4(D) is a longitudinal sectional diagram of the second example taken along the line IVD-IVD of FIG. 4(C). FIG. 4(E) is a cross sectional diagram showing the first example when worn (before absorbing body fluid), and FIG. 4(F) is a cross sectional diagram showing the first example after absorbing the body fluid.

In the first example as shown in FIGs. 4(A) and 4(B), a leak preventer 13 has pleats on outer side surfaces of bags 31. In the second example as shown in FIGs. 3(C) and 3(D), a leak preventer 14 has pleats on rear side surfaces of bags 32.

As described above, absorbers are provided in each of the two bags, and the absorbers absorb a body fluid. Thus, an absorption volume of the body fluid in the absorbers is also defined by a volume of the bags. The volume of the bags is determined taking in consideration a volume of the absorbers before (dry) and after (swollen) use. Thus, as the two examples shown in FIGs. 4, pleats are provided to the bags so that the volume of the bags can be small before use and large after use. To be specific, states of the first example when worn (before absorbing body fluid) and the first example after absorbing the body fluid are shown in FIGs. 4(E) and 4(F), respectively.

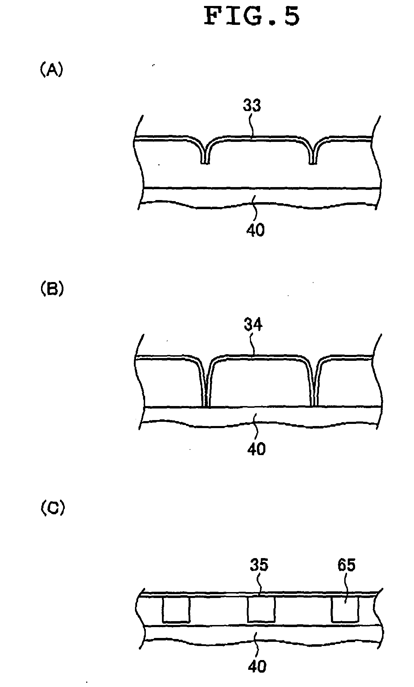

FIGs. 5 are schematic diagrams each showing a leak preventer and an absorber having a space between an inner surface of a bag of the leak preventer and an upper surface of the absorber. FIG. 5(A) is a partial cross sectional diagram showing a first example, FIG. 5(B) is a partial cross sectional diagram showing a second example, and FIG. 5(C) is a partial cross sectional diagram showing a third example.

In the first example as shown in FIG. 5(A), a bag 33 of a leak preventer is partially folded toward the absorber 40 to form a space therebetween.

In the second example as shown in FIG. 5(B), a bag 34 of a leak preventer is partially folded toward the absorber 40 to form a space therebetween. The difference from the first example is that tips of the folded portions of the bag 34 are in contact with the absorber 40.

In the third example as shown in FIG. 5(C), a spacer 65 is provided between a bag 35 and the absorber 40 of a leak preventer to form a space. A foamed product is preferably used as the spacer 65 for bulkiness.

A distance between an inner surface of the bag of the leak preventer and an upper surface of the absorber is preferably 0.5 to 10 mm.

FIGs. 6 are schematic cross sectional diagrams showing other examples of the absorbent article of the present invention. In FIGs. 6, structural members other than the leak preventer, the absorbers, and the fitting member are omitted.

An absorbent article 110 shown in FIG. 6(A) is provided with: a leak preventer 17 having a sheet 21 and two bags 30 formed separately on right and left of an upper side of the sheet 21, each of the two bags having an opening facing with each other; the absorbers 40each having three layers which contain a super absorbent polymer and may absorb a body fluid, provided in the respective two bags 30; and the fitting member 70.

In the absorbent article 110, the sheet 21 of the leak preventer 17 is folded. In the absorbent article 110, the two bags 30 of the leak preventer 17 are independent of each other.

The absorbent article 110 is preferably provided with a guide sheet allowing transfer of a body fluid between the absorbers.

An absorbent article 120 shown in FIG. 6(B) is provided with a connecting absorber 45 on an upper side of the sheet 21 for connecting the absorbers 40 provided in the respective two bags 30, in addition to the structure of the absorbent article 110 shown in FIG. 6(A).

The absorbent article 120 allows transfer of a body fluid between the absorbers 40 through the connecting absorber 45. A super absorbent sheet of one layer is preferably used as the connecting absorber 45 for suppressing discomfort at the crotch of the body when the absorbers swell. Further, a nonwoven fabric or the like having a function of transferring a body fluid is preferably laminated to the connecting absorber 45.

An absorbent article 130 shown in FIG. 6(C) is provided with a connecting absorber 45 inside a connecting portion 47 connecting the two bags 30 at a lower side of the sheet 21, for connecting the absorbers 40 provided in the respective two bags 30, in the structure of the absorbent article 110 shown in FIG. 6(A).

The absorbent article 130 allows transfer of a body fluid between the absorbers 40 through the connecting absorber 45. A super absorbent sheet of one layer is preferably used as the connecting absorber 45 for suppressing discomfort at the crotch of the body when the absorbers swell.

An absorbent article 135 shown in FIG. 6(D) is provided with a connecting portion 48 which does not make a barrier between a lower surface of the fitting member 70 and an upper surface of the connecting absorber 45 instead of the connecting portion 47, in addition to the structure of the absorbent article 130 shown in FIG. 6(C).

The absorbent article 135 is capable of transferring a body fluid between the absorbers 40 through the connecting absorber 45. A super absorbent sheet of one layer is preferably used as the connecting absorber 45 for suppressing discomfort at the crotch of the body when the absorbers swell.

### <Guide sheet>

Examples of a preferable guide sheet include a sheet member having a grooved structure, a concavity-and-convexity-containing sheet member, and a nonwoven fabric sheet member. The sheet member may be used alone, or in combination of two or more thereof.

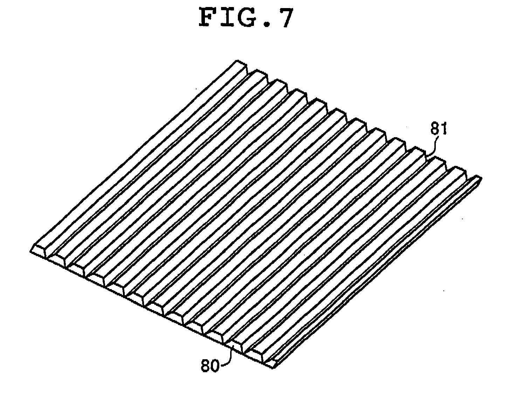

FIG. 7 is a schematic perspective view showing an example of a sheet member having a grooved structure. A sheet member 80 has a grooved structure 81, and the grooved structure 81 serves as flow paths of a body fluid.

A sheet member having a grooved structure is obtained through a method of providing a grooved structure serving as flow paths on a thermoplastic and body fluid impermeable film by thermoforming.

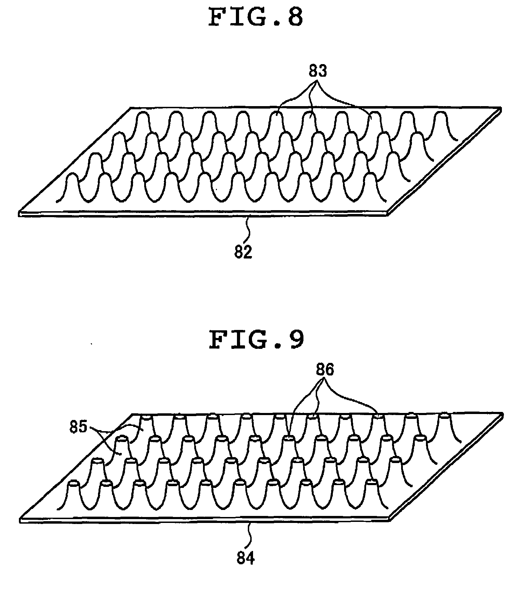

FIG. 8 is a schematic perspective view showing an example of a concavity-and-convexity-containing sheet member. A concavity-and-convexity-containing sheet member 82 shown in FIG. 8 has many protrusions 83 forming convex portions.

The concavity-and-convexity-containing sheet member has at least one uneven surface having concave portions and convex portions. In the concavity-and-convexity-containing sheet member, many continuous concave portions serve as flow paths of a body fluid. The concavity-and-convexity-containing sheet member has an advantage in that even when some convex portions are deformed in use, transfer of a body fluid is not inhibited.

An example of a material for the concavity-and-convexity-containing sheet member includes a body fluid impermeable film composed of a resin such as PE, PP, PVA, or urethane.

In the concavity-and-convexity-containing sheet member, part of or all of the convex portions may be partially or entirely provided with apertures.

FIG. 9 is a schematic perspective view showing an example of a concavity-and-convexity-containing sheet member having all of the convex portions provided with apertures (hereinafter, referred to as "apertured sheet member"). An apertures-provided sheet member 84 shown in FIG. 9 has many protrusions 85 forming convex portions, and the protrusions 85 have apertures 86 on respective vertices thereof. In the apertures-provided sheet member as shown in FIG. 9, the apertures also serve as flow paths of a body fluid. The apertures-provided sheet member shown in FIG. 9 is preferably used with a surface having larger apertures on a wearer side.

A specific example of the apertures-provided sheet member includes one suggested by the inventors of the present invention in WO 02/065965.

When an apertures-provided sheet member is used as a guide sheet, a body fluid may remain in apertures causing a liquid residue, that is, a rewetting value may become worse. In this case, a hydrophilic diffusion sheet is preferably used in combination under a lower side of the apertures-provided sheet member. Thus, a body fluid present in the apertures permeates and transfers through the hydrophilic diffusion sheet, to thereby drastically decrease a rewetting value. Further, the apertures-provided sheet member is prevented from shifting from the leak preventer, and its form stability improves as well.

A nonwoven fabric product having a relatively light weight is preferably used as the hydrophilic diffusion sheet. Examples of the hydrophilic nonwoven fabric product include: tissue paper containing rayon, cotton, wood pulp, or the like; and a hydrophilic nonwoven fabric. Specific examples thereof include: tissue paper; a rayon spun bond (TCF, available from Futamura Chemical Co., Ltd., for example); a cotton spun lace, a rayon/PP mixed spun lace; and rayon/PET mixed spun lace.

Further examples thereof include: a spun bond nonwoven fabric of PP, PE, or PET; a thermal bond nonwoven fabric of PP; and a nonwoven fabric subjected to hydrophilic treatment through surface treatment with a surfactant or the like of a synthetic nonwoven fabric such as a through-air nonwoven fabric of PE/PET.

The apertures-provided sheet member and the hydrophilic diffusion sheet may be laminated through simple superposition or may be combined through hot melting, heat laminating, or the like.

A body fluid impermeable sheet is preferably used in combination under the hydrophilic diffusion sheet. Thus, the body fluid is not even temporarily accumulated between the hydrophilic diffusion sheet and the sheet of the leak preventer and is quickly transferred to bags of the leak preventer. An example of a material for the body fluid impermeable sheet includes a film made of a resin such as PE, PP, PVA, or polyurethane.

The hydrophilic diffusion sheet and the body fluid impermeable sheet may be laminated through simple superposition or may be combined through hot melting, heat laminating, or the like.

The apertures-provided sheet member and the body fluid impermeable sheet may be used in combination instead of using the hydrophilic diffusion sheet.

The apertures-provided sheet member and the body fluid impermeable sheet may be laminated through simple superposition or may be combined through hot melting, heat laminating, or the like.

Inner spaces of the nonwoven sheet member serve as flow paths of a body fluid. The nonwoven sheet member preferably has large compressive resistance (so-called resilience). A nonwoven fabric obtained by integrally laminating a paper layer or nonwoven fabric layer having a smooth surface and a thick fibrous web having a bulky and uneven surface, which is proposed by the inventors of the present invention in JP 2003-103704 A and JP 2003-103677 A can be preferably used.

Next, arrangements of a guide sheet will be described.

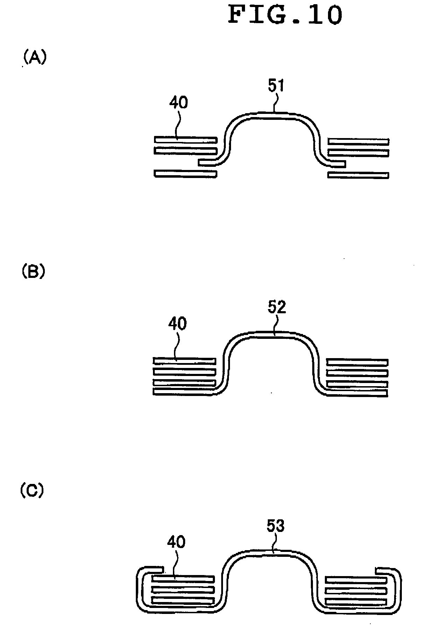

FIGs. 10 are schematic cross sectional diagrams explaining various arrangements of a guide sheet. In FIGs. 10, structural members other than the absorbers and the guide sheet are omitted.

In FIG. 10(A), right and left edges of a guide sheet 51 extend to areas between a second layer and a third layer (from top) of the absorbers 40 each consisting of three layers.

In FIG. 10(B), right and left edges of a guide sheet 52 extend to entirely cover a lower surface of a third layer (from top) of the absorbers 40 each consisting of three layers. As described above, a preferable aspect includes the guide sheet extending to partially or entirely cover a lower surface of the absorbers. In this aspect, a body fluid is efficiently absorbed even in outer edge portions of the absorbers.

In FIG. 10(C), right and left edges of a guide sheet 53 extend to entirely cover a lower surface of a third layer (from top) of the absorbers 40 each consisting of three layers and a side surface thereof. As described above, a preferable aspect includes the guide sheet extending to partially or entirely cover a side surface of the absorbers, in addition to a lower surface of the absorbers. In this aspect, a body fluid is efficiently absorbed even in an outer edge portion on an upper portion of the absorbers.

### <Structure of body fluid receiving region>

In the present invention, the term "body fluid receiving portion" refers to a portion between two bags of a leak preventer.

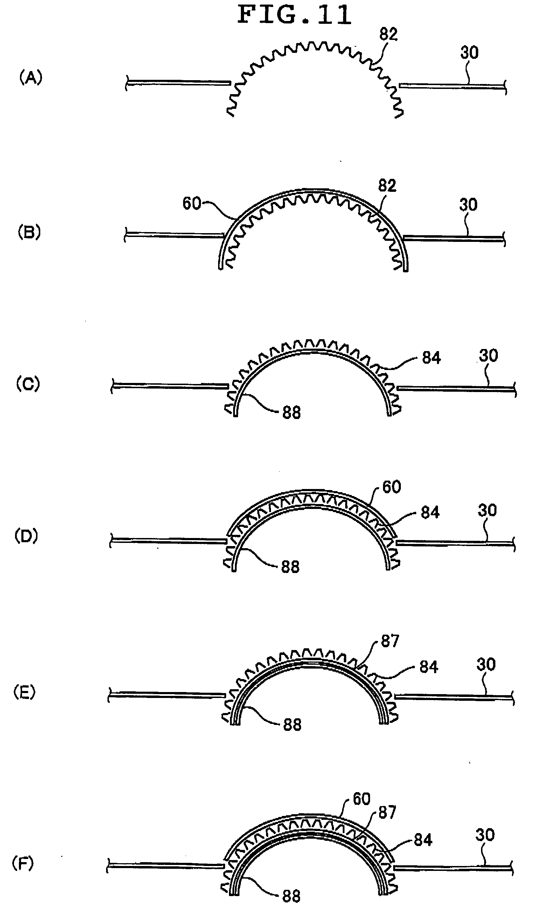

FIGs. 11 are schematic partial cross sectional diagrams showing various structures of a body fluid receiving region of the absorbent article of the present invention.

FIG. 11(A) shows an absorbent article having the concavity-and-convexity-containing sheet member 82 without apertures for serving as a guide sheet, exposed in a body fluid receiving region.

FIG. 11(B) shows an absorbent article having the skin contact sheet 60 on an upper side of the concavity-and-convexity-containing sheet member 82 of the absorbent article of FIG. 11(A).

FIG. 11(C) shows an absorbent article having a combination of the apertures-provided sheet member 84 serving as a guide sheet and the body fluid impermeable sheet 88 under a lower surface of the apertures-provided sheet member 84, exposed in a body fluid receiving region.

FIG. 11(D) shows an absorbent article having the skin contact sheet 60 on an upper side of the apertures-provided sheet member 84 of the absorbent article of FIG. 11(C).

FIG. 11(E) shows an absorbent article having a combination of the apertures-provided sheet member 84 serving as a guide sheet, the hydrophilic diffusion sheet 87 laminated under a lower surface of the apertures-provided sheet member 84, and the body fluid impermeable sheet 88 laminated under a lower surface of the hydrophilic diffusion sheet 87, exposed in a body fluid receiving region.

FIG. 11(F) shows an absorbent article having the skin contact sheet 60 on an upper side of the apertures-provided sheet member 84 of the absorbent article of FIG. 11(E).

In the absorbent article of the present invention, a body fluid is discharged to a body fluid receiving portion closely-contacting with a urethral meatus of a wearer and is transferred to bags without accumulating therein.

In this case, the absorbent article preferably has a function of receiving a large volume of body fluid in a very short period of time. Usually, a healthy child discharges 100 mL of urine in about 20 seconds. A healthy adult discharges urine at a rate equal to or higher than the above rate. Thus, even if 100 mL of urine is discharged in 15 seconds or less, preferably 10 seconds or less, the urine is preferably not accumulated in the body fluid receiving region or not transferred to an outer surface of the absorbent article or the underwear.

Thus, the body fluid receiving region preferably has no pulp or SAP, or at most has a small amount thereof.

In order to facilitate transfer of a body fluid by gravity, the center of a sheet member of a leak preventer is preferably arranged at a high position so that a body fluid is discharged thereto.

Further, in order to prevent transfer of a body fluid to an outer surface of the absorbent article or underwear, the close contact between a urethral meatus and a body fluid receiving region is preferably increased. To be specific, a member exposed in a body fluid receiving region has a certain thickness, preferably 0.5 mm or more, more preferably 1.0 mm or more, to thereby exert cushioning properties. A fitting member is preferably used to increase the close contact between the urethral meatus and the body fluid receiving region. Further, such techniques may be combined.

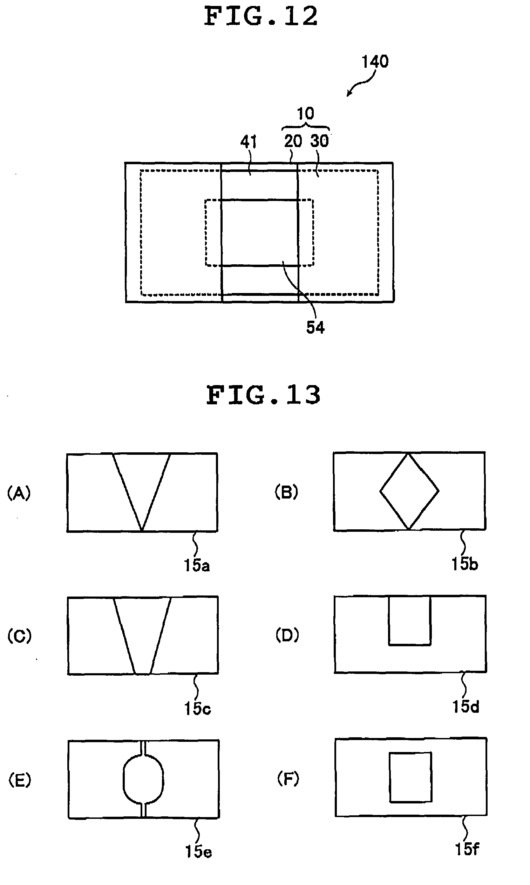

FIG. 12 is a schematic plan view showing another example of the absorbent article of the present invention. In FIG. 12, structural members other than the leak preventer, the absorbers, and the guide sheet are omitted.

An absorbent article 140 shown in FIG. 12 is provided with: the leak preventer 10 having the sheet 20 and the two bags 30; absorbers 41 integrally provided in and between the two bags 30; and a guide sheet 54 provided with spaces on front and rear sides of the article.

In the absorbent article 140, SAP is present in the body fluid receiving portion, but a body fluid quickly transfers to the bags 30 through the guide sheet 54.

A shape of the body fluid receiving region is not particularly limited, and may be a shape other than the rectangular shape shown in FIGs. 1 and the like.

FIGs. 13 are schematic plan views showing various examples of a leak preventer of the absorbent article of the present invention.

A leak preventer 15a shown in FIG. 13(A) has apertures of the two bags in contact with each other on a rear side, and has a triangular body fluid receiving region.

A leak preventer 15b shown in FIG. 13(B) has apertures of the two bags in contact with each other on front and rear sides, and has a rhombus body fluid receiving region.

A leak preventer 15c shown in FIG. 13(C) has apertures of the two bags with a wide space therebetween on a front side and a narrow space therebetween on a rear side, and has a trapezoid body fluid receiving region.

A leak preventer 15d shown in FIG. 13(D) has apertures of the two bags connected on a rear side (partially cutaway type).

A leak preventer 15e shown in FIG. 13(E) has apertures of the two bags with a wide space therebetween at the center and a very narrow space therebetween on edges (center-type).

A leak preventer 15f shown in FIG. 13(F) has apertures of the two bags connected on front and rear sides (window-type).

The size of the body fluid receiving region is not particularly limited, but a rectangular body fluid receiving region preferably has a front-to-rear length of 5 to 20 cm and a right-to-left length of 2 to 10 cm.

An area of the body fluid receiving region is preferably 50% or less of an area of the entire sheet. The area in the above range leads to relatively large areas of the bags, to thereby increase an absorption volume of the body fluid.

### <Size, shape, or the like of absorbent article>

The absorbent article of the present invention is used to be positioned at a front part of a diaper or the underwear worn by a wearer for receiving urine and feces separately.

Thus, the absorbent article of the present invention preferably has a size which fits into the front part thereof. To be specific, an absorbent article for a child preferably has a surface area of 200 cm² or less, and an absorbent article for an adult preferably has a surface area of 400 cm² or less. An absorbent article is preferably thin for better wearing feel.

Meanwhile, a sufficient absorption volume of a body fluid must be secured. To be specific, the volume is 200 mL or more for a child, and 300 mL or more for an adult.

In consideration of the above, the absorbent article of the present invention preferably has a surface area of 50 to 150 cm² for a child, and 80 to 300 cm² for an adult. An average front-to-rear length L thereof is preferably 20 cm or less. Further, a thickness thereof for a child or adult is preferably about 1 to 5 mm.

A conventional child diaper with a body fluid absorption volume of 200 mL or more has a surface area of 200 cm² or more and a thickness of 5 mm or more, and a conventional adult diaper with a body fluid absorption volume of 300 mL or more has a surface area of 350 cm² or more and a thickness of 5 mm or more. Thus, the absorbent article of the present invention has a smaller surface area and a smaller thickness compared with those of the conventional diapers.

The shape of the absorbent article is not particularly limited.

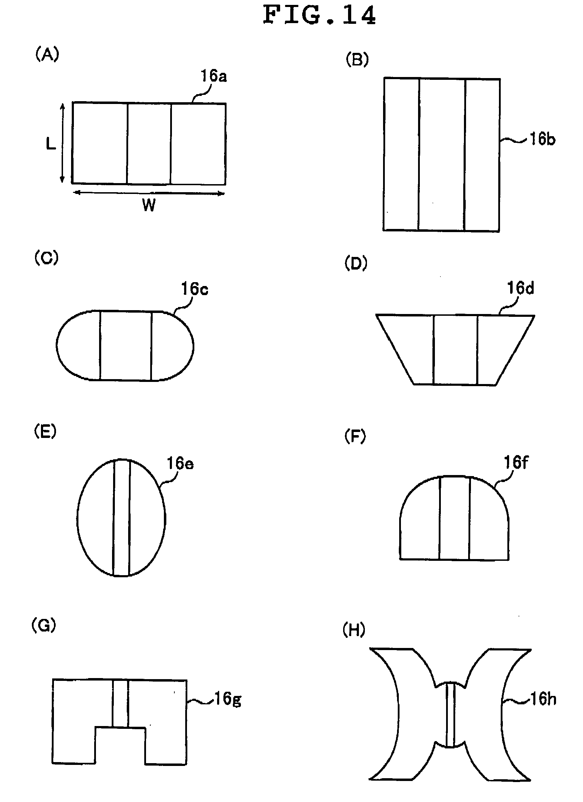

FIGs. 14 are schematic plan views showing various examples of a leak preventer of the absorbent article of the present invention.

A leak preventer 16a shown in FIG. 14(A) has a rectangular shape with a longer right-to-left (average) length W than a front-to-rear (average) length L.

A leak preventer 16b shown in FIG. 14(B) has a rectangular shape with a shorter right-to-left (average) length W than a front-to-rear (average) length L.

A leak preventer 16c shown in FIG. 14(C) has a shape obtained by rounding 4 corners of the rectangle of the leak preventer 16a shown in FIG. 14(A) and by providing a wider space between the apertures of the bags.

A leak preventer 16d shown in FIG. 14(D) has a trapezoid shape obtained by cutting off 2 corners (on rear side) of the rectangle of the leak preventer 16a shown in FIG. 14(A).

A leak preventer 16e shown in FIG. 14(E) has a shape obtained by rounding 4 corners of the rectangle of the leak preventer 16b shown in FIG. 14(B) and by providing a narrow space between the apertures of the bags.

A leak preventer 16f shown in FIG. 14(F) has a shape obtained by rounding 2 corners (on front side) of the rectangle of the leak preventer 16a shown in FIG. 14(A).

A leak preventer 16g shown in FIG. 14(G) has a shape obtained by providing large side portions (on rear side) of the bags of the leak preventer 16a shown in FIG. 14(A) and by providing a narrow space between the apertures of the bags.

A leak preventer 16h shown in FIG. 14(H) has a shape obtained by providing large side portions (on front and rear sides) of the bags of the leak preventer 16a shown in FIG. 14(A) and by providing a narrow space between the apertures of the bags.

The absorbent article of the present invention has a ratio of a right-to-left average length W to a front-to-rear average length L (W/L) of preferably 0.5 or more, more preferably 1.0 or more, furthermore preferably 1.2 or more. The ratio in the above range provides better wearing feel.

### <Fitting member>

A fitting member serves to fit a body fluid receiving region of an absorbent article to a body surface region. A member having elasticity (cushioning properties) or stretchability is preferably used as the fitting member.

Examples of the member having elasticity include: a synthetic rubber sheet having a relatively low hardness; an elastic tube; a foamed product (made of PU, PP, or PP/EVA, for example); and a bulky nonwoven fabric having high resilience. The member having elasticity allows close-contact of the body fluid receiving region of the absorbent article to the body surface region due to its rebound resilience.

Examples of the member having strechability include a stretchable rubber tape, a urethane knitted filament, and stretchable urethane foam. The member having stretchability allows close-contact of the body fluid receiving region of the absorbent article to the body surface region by stretching in a front-to-rear direction so that an upward force is applied to a portion where the body fluid receiving region of the absorbent article and the body surface region are in contact with each other.

The following three arrangement aspects of the fitting member can be used: an aspect of arranging a fitting member on an upper side of a sheet of a leak preventer; an aspect of arranging a fitting member under a lower side of a sheet of a leak preventer; and an aspect of arranging fitting members both on upper side and under lower side of a sheet of a leak preventer.

In any aspect, the fitting member may be bonded to a leak preventer and/or a guide sheet. Examples of a bonding method include a method using an adhesive, a hot-melt adhesive, or a face fastener.

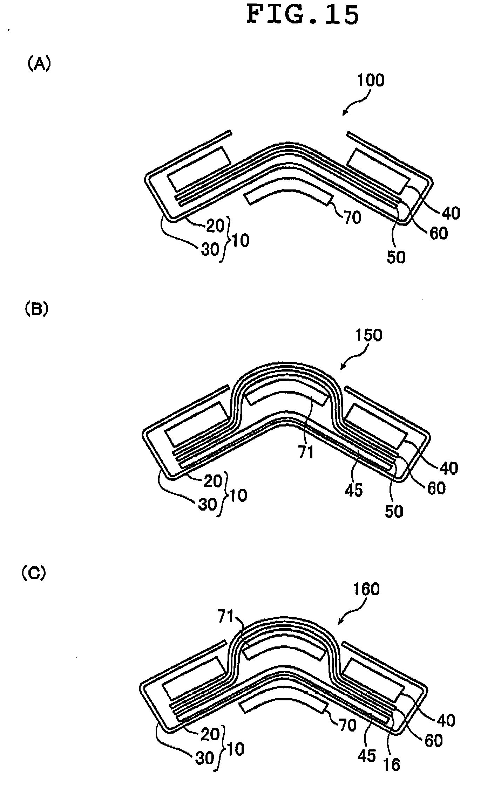

FIGs. 15 are schematic cross sectional diagrams showing various examples of the absorbent article of the present invention.

The absorbent article 100 shown in FIG. 15(A) is the same as the absorbent article 100 shown in FIGs. 1. In the absorbent article 100, the fitting member 70 is arranged under a lower side of the sheet 20 of the leak preventer 10.

An absorbent article 150 shown in FIG. 15(B) is the same as the absorbent article 100 except that a fitting member 71 is arranged above an upper side of the sheet 20 of the leak preventer 10 instead of the fitting member 70 and that the connecting absorber 45 is provided under a lower side of the guide sheet 50. When the fitting member is arranged above an upper side of the sheet of the leak preventer as described above, the fitting member is preferably body fluid impermeable to allow quick transfer of the body fluid to the bags.

An absorbent article 160 shown in FIG. 15(C) is the same as the absorbent article 150 except that the fitting member 70 is arranged under a lower side of the sheet 20 of the leak preventer 10.

The present invention may employ an aspect in which a leak preventer is provided with a penetration path and a fitting member penetrates the leak preventer therethrough.

A right-to-left width of the fitting member is not particularly limited as long as the fitting member can serve to fit the body fluid receiving region of the absorbent article to the body surface region including a urethral meatus.

A front-to-rear length of the fitting member is not particularly limited as long as the fitting member can serve to fit the body fluid receiving region of the absorbent article to the body surface region including a urethral meatus. Two preferable aspects thereof are: an aspect in which the fitting member has substantially the same front-to-rear length as that of the leak preventer; and an aspect in which the fitting member has a longer front-to-rear length than that of leak preventer (including an aspect in which the fitting member stretches in use to become longer than the leak preventer).

The aspect in which the fitting member has substantially the same front-to-rear length as that of the leak preventer is suitably used when the fitting member is a member having elasticity.

Meanwhile, the aspect in which the fitting member has a longer front-to-rear length than that of the leak preventer is suitably used when at least part of the fitting member is a member having stretchability (hereinafter, referred to as "stretchable fitting member").

When the front-to-rear length of the fitting member is longer than the front-to-rear length of the leak preventer, a ratio or lengths of portions protruding toward a front side and a rear side of the leak preventer are not particularly limited, and are arbitrarily adjusted depending on the purpose.

The fitting member may include a combination of 2 or more types of members.

For example, when the front-to-rear length of the fitting member is longer than the front-to-rear length of the leak preventer, a portion protruding from the leak preventer may be a stretchable fitting member and a portion brought into contact with the leak preventer may be a member having elasticity. The stretchable fitting member and the member having elasticity may be bonded and used.

Further, a member having elasticity may be provided in a portion in contact with the leak preventer, and a stretchable fitting member may be provided over the entire length of a portion protruding from the leak preventer and a portion in contact with the leak preventer under a lower side or above an upper side of the member having elasticity, to provide a double-layer structure to the portion in contact with the leak preventer. In this case, as in the absorbent article 160 shown in FIG. 15(C), the fitting member 71 and the fitting member 70 may be provided above an upper side and under a lower side of the sheet 20 of the leak preventer 10, respectively. The fitting member 71 and the fitting member 70 may be constructed of a member having elasticity and a stretchable fitting member, respectively.

When using the fitting member having a longer front-to-rear length than the front-to-rear length of the leak preventer (hereinafter, referred to as "extended fitting member"), preferable aspects to be used are: an aspect in which the absorbent article of the present invention is detachably attached to a cover (underwear, shorts, and diaper cover, for example) to be used; and an aspect in which the absorbent article of the present invention is provided with a cover portion, and fixed to the cover portion to be used (disposable diaper, for example). In any aspect, the fitting member has an effect of preventing the absorbent article from being dislocated greatly by movement of a body of a wearer.

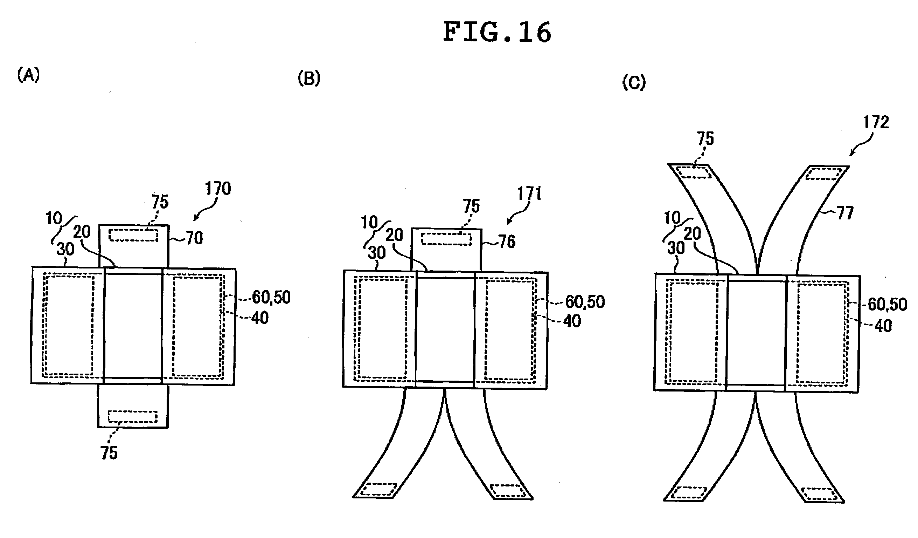

FIGs. 16 are schematic plan views showing various examples of the absorbent article of the present invention having a detachable member for a fitting member.

An absorbent article 170 shown in FIG. 16(A) is provided with a detachable member 75 on back sides of both edges of the fitting member 70 of the absorbent article 100 shown in FIGs. 1. Examples of the detachable member 75 include a hook-and-loop fastener and an adhesive. Thus, by providing the detachable member, the absorbent article of the present invention may be detachably attached to a cover to be used.

An absorbent article 171 shown in FIG. 16(B) is provided with a fitting member 76 having edge portions as split into two lengs on a rear side instead of the fitting member 70 of the adsorbent article 170 shown in FIG. 16(A). The edge portions each have the detachable member 75.

An absorbent article 172 shown in FIG. 16(C) is provided with a fitting member 77 having edge portions as split into two lengs on front and rear sides instead of the fitting member 70 of the absorbent article 170 shown in FIG. 16(A). The edge portions each have the detachable member 75.

When the fitting member is split and the detachable member is provided in each of the edge portions as in the absorbent article 171 shown in FIG. 16(B) and the absorbent article 172 shown in FIG. 16(C), a position of the absorbent article when worn is further stabilized. Further, when the edge portions on a rear side are branched, it is easy to prevent the fitting member from covering the anus, to further facilitate separate receiving of urine and feces.

The number of split edge portions is not particularly limited.

The use of a plurality of fitting members instead of splitting one fitting member can provide the same effects as the splitting even when the number of detachable members is increased.

### <Absorbent article set>

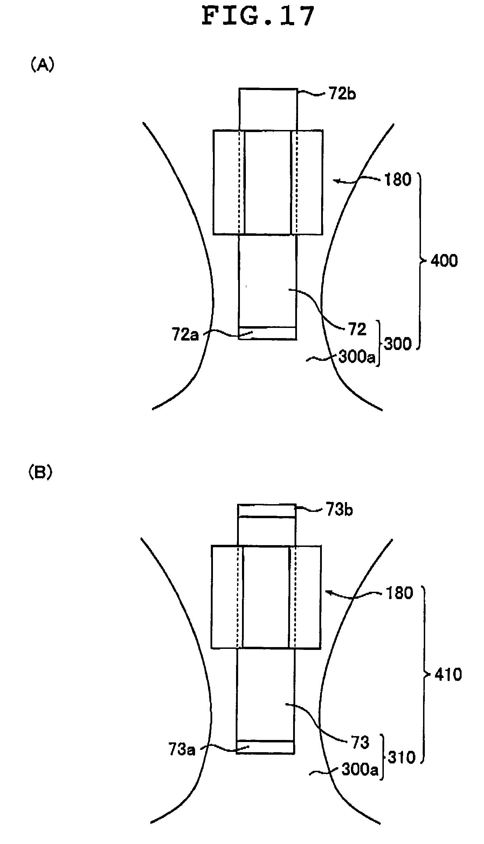

FIGs. 17 are partial plan views schematically showing various examples of an absorbent article set of the present invention which consists of the absorbent article of the present invention and a cover. In FIGs. 17, only a cover at the crotch is shown.

An absorbent article set 400 shown in FIG. 17(A) consists of an absorbent article 180 of the present invention and a cover 300. The absorbent article 180 is the same as the absorbent article 100 except that the fitting member 70 is not included. The cover 300 has a cover main body 300a and a fitting member 72. The fitting member 72 is a stretchable fitting member and has a rear edge portion 72a fixed substantially at the center of an upper side of the crotch of the cover 300 and an unfixed front edge portion 72b. A detachable member that is not shown in the figure is provided in the front edge portion 72b. The rear edge portion 72a may be detachable.

The absorbent article set 400, when worn, is used by placing the absorbent article 180 at an appropriate position on an upper side of the fitting member 72 of the cover 300 and fixing the front edge portion 72b of the fitting member 72 to an appropriate position of the cover main body 300a so that an appropriate force is applied in a direction of a body surface. In this case, the absorbent article 180 and the fitting member 72 may be fixed by a hook-and-loop fastener or the like.

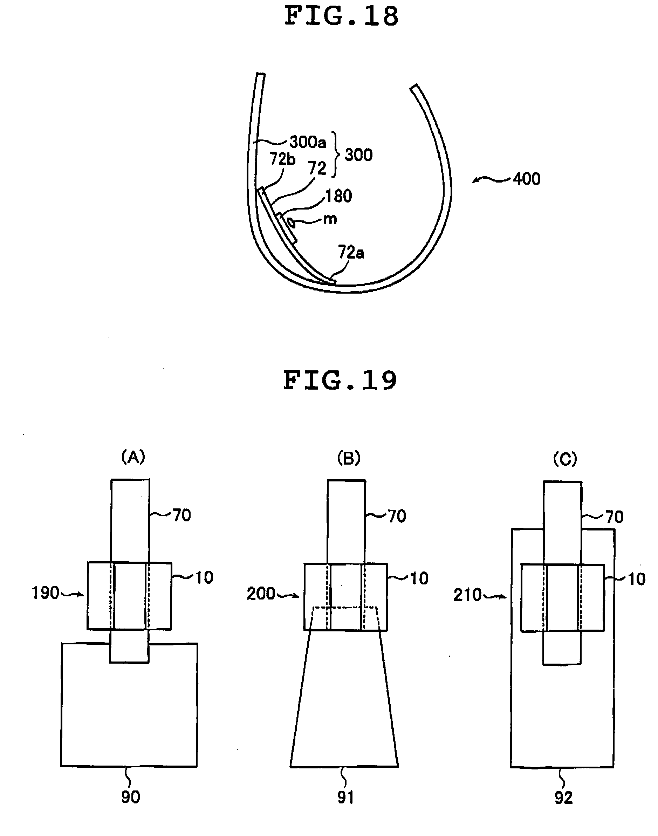

FIG. 18 is a longitudinal sectional diagram of the absorbent article set shown in FIG. 17(A) taken along the face passing the crotch of the body when worn. As shown in FIG. 18, the absorbent article 180 fits to the body surface region including the urethral meatus m by the function of the fitting member 72 of the cover 300.

An absorbent article set 410 shown in FIG. 17(B) consists of the absorbent article 180 of the present invention and a cover 310. The cover 310 has the cover main body 300a and a fitting member 73. The fitting member 73 is a stretchable fitting member and has a rear edge portion 73a fixed substantially at the center of an upper side of the crotch of the cover 310 and a front edge portion 73b fixed inside a front part of the cover 310.

The absorbent article set 410, when worn, is used by placing the absorbent article 180 at an appropriate position on an upper side of the fitting member 73 of the cover 310. In this case, the absorbent article 180 and the fitting member 73 may be fixed by a hook-and-loop fastener or the like.

### <Feces-receiving portion>

A preferable aspect of the absorbent article of the present invention is further provided with a feces-receiving portion.

FIGs. 19 are schematic plan views showing various examples of the absorbent article of the present invention having a feces-receiving portion.

An absorbent article 190 shown in FIG. 19(A) is the same as the absorbent article 100 shown in FIGs. 1 except that a feces-receiving portion 90 is further provided. The feces-receiving portion 90 has the same structure as that of a conventionally known diaper. The feces-receiving portion 90 is fixed at a rear edge portion of the fitting member 70. In the absorbent article 190, the urine and the feces are received completely separately in a front part of the absorbent article 190 and in the feces-receiving portion 90 in a rear part thereof, respectively.

An absorbent article 200 shown in FIG. 19(B) is the same as the absorbent article 100 shown in FIGs. 1 except that a feces-receiving portion 91 is provided. The feces-receiving portion 91 has a structure in which the front edge portion of the feces-receiving portion 90 shown in FIG. 19(A) is extended to the crotch to overlap the rear edge portion of the body fluid receiving region onto a lower surface thereof. In the absorbent article 200, the urine is received in a front part of the absorbent article 200 and the feces is received in the feces-receiving portion in a rear part thereof. However, when the urine partially leaks from the front part because of a posture of the wearer or the like, the urine can be received in the feces-receiving portion 91.

An absorbent article 210 shown in FIG. 19(C) is the same as the absorbent article 100 shown in FIGs. 1 except that a feces-receiving portion 92 is provided. The feces-receiving portion 92 has a structure in which the front edge portion of the feces-receiving portion 90 shown in FIG. 19(A) is extended to the front part to overlap the entire area of the leak preventer 10 onto a lower surface thereof. In the absorbent article 210, the urine is received in a front part of the absorbent article 210 and the feces is received in the feces-receiving portion in a rear part thereof. However, when the urine partially leaks from the front part because of a posture of the wearer or the like, the urine can be received in the feces-receiving portion 92.

### <Relationship with posture>

The absorbent article of the present invention can absorb more body fluid than the conventional absorbent article in any posture as confirmed by the following body fluid absorption test.

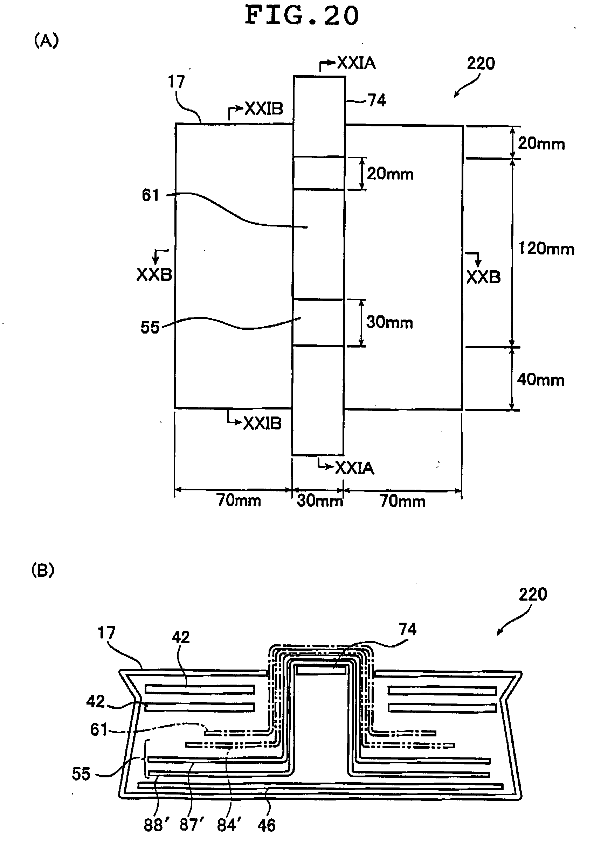

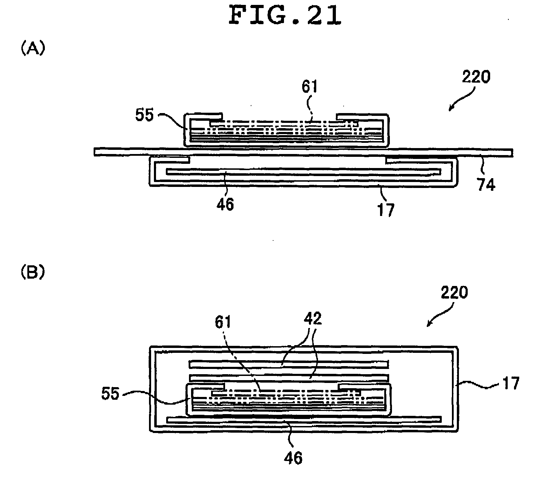

FIGs. 20 and 21 are schematic diagrams each showing an absorbent article of the present invention used for a body fluid absorption test. FIG. 20(A) is a plan view, and FIG. 20(B) is a cross sectional diagram taken along the line XXB-XXB of FIG. 20(A). FIG. 21(A) is a longitudinal sectional diagram taken along the line XXIA-XXIA of FIG. 20(A), and FIG. 21 (B) is a longitudinal sectional diagram taken along the line XXIB-XXIB of FIG. 20(A).

An absorbent article 220 shown in FIGs. 20 is provided with: a leak preventer 17; two-layered absorbers 42 provided in the respective two bags of the leak preventer 17; a skin contact sheet 61; a guide sheet 55; a fitting member 74; and a connecting absorber 46. The absorbent article 220 is the same as the absorbent article 135 shown in FIG. 6(D) except that the structure and the shape of the body fluid receiving region shown in FIG. 11(F) and FIG. 13(F) are employed.

The guide sheet 55 will be described in more detail.

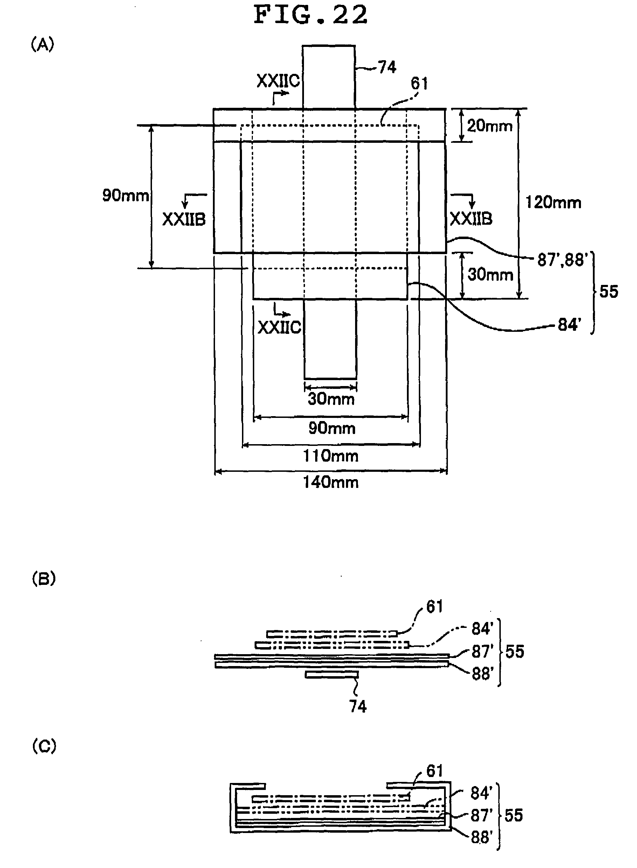

FIGs. 22 are schematic diagrams each showing a skin contact sheet, a guide sheet, and a fitting member of the absorbent article of the present invention used for a body fluid absorption test. FIG. 22(A) is a plan view. FIG 22(B) is a cross sectional diagram taken along the line XXIIB-XXIIB of FIG. 22(A), and FIG. 22(C) is a longitudinal sectional diagram taken along the line XXIIC-XXIIC of FIG. 22(A).

As shown in FIG. 22(B), the guide sheet 55 is provided with: an apertures-provided sheet member 84'; a hydrophilic diffusion sheet 87' laminated under a lower surface of the apertures-provided sheet member 84'; a body fluid impermeable sheet 88' laminated under a lower surface of the hydrophilic diffusion sheet 87'; and a skin contact sheet 61 laminated above an upper side of the apertures-provided sheet member 84'. The body fluid impermeable sheet 88' is folded at a front edge portion and a rear edge portion to cover a front edge portion and a rear edge portion of the skin contact sheet 61. A fitting member 74 is provided longitudinally at the center of a lower surface of the guide sheet 55. A planar shape of each of the members is shown in FIG. 22(A). The skin contact sheet 61, the hydrophilic diffusion sheet 87', and the body fluid impermeable sheet 88' are cut away in rear edge portions of both side portions, and each has a T shape. This prevents transfer of the urine to a rear part along the guide sheet 55.

A composite sheet of a polyethylene film and a polypropylene nonwoven fabric was used as the leak preventer 17.

A highly absorbent sheet prepared by coating SAP-dispersed slurry on a polyester nonwoven fabric (MegaThin, available from Japan Absorbent Technology Institute, SAP amount: 180 g/m²) was used for the absorbers 42 and the connecting absorber 46.

A composite sheet including the polyethylene apertures-provided sheet member 84' (available from Tredegar Film Products), the hydrophilic diffusion sheet 87' made of TCF (available from Futamura Chemical Co., Ltd.), and the body fluid impermeable sheet 88' of a PE film from the top was used as the guide sheet 55.

An air-through nonwoven fabric (available from Kuraray Co., Ltd.) was used as the skin contact sheet 61.

An extended fitting member of stretchable urethane foam (available from Inoac Corporation) was used as the fitting member 74.

The size of each of the members is shown in FIG. 20(A) and FIG. 22(A).

Next, a body fluid absorption test was carried out in various postures using the absorbent article of the present invention.

The body fluid absorption test was carried out by: attaching the absorbent article on a human body model (manufactured by Hagoromo Mannequin Co., Ltd.) having a hole at a position corresponding to a urethral meatus; and discharging out a physiological saline from the hole of the human body model in postures of standing, sitting, lying face-down, lying on side, and lying on back. The discharge was carried out by 100 mL with 5-minute intervals and continued until a leak from the absorbent article was observed, to thereby calculate a total absorption volume until the leak.

The same body fluid absorption test was carried out using a commercially available incontinence pad ("Lifree Anshin Nyoutori Super Strength Pads", available from Unicharm Corporation) as the conventional absorbent article.

Table 1 shows the results and design absorption volumes of the respective absorbent articles.

The design absorption volume refers to a free swelling capacity, a retention capacity, and AUL.

The free swelling capacity was measured through the following procedure. A weight of an absorbent article was measured in advance, and the absorbent article was immersed in a 0.9 wt% aqueous solution of sodium chloride for 30 minute. Then, the pad was pulled out and maintained in a pulled-out state for 30 seconds to drain water therefrom. The pad was placed on a metal mesh for draining so that the skin contact sheet was positioned to a lower side. An acrylic sheet was placed thereon to apply a load on the entire absorber, and a weight (10 kg) was further placed thereon. The resultant was left to stand for 20 minutes to drain water therefrom. Then, a weight of the pad was measured, to thereby determine a weight of the pad increased from that before absorption as a free swelling capacity (specific gravity of an aqueous solution of sodium chloride was 1). The measurement was carried out at n = 2.

The retention capacity was measured through the following procedure. The sample used for measuring the free swelling capacity was placed in a dehydration basket of a dehydrator (150 G) so that a side of the skin contact sheet touched a side surface of the dehydration basket, and was dehydrated for 90 seconds. Then, a weight of the sample was measured, to thereby determine a weight of the sample increased from that before absorption as the retention capacity (specific gravity of an aqueous solution of sodium chloride was 1). The measurement was carried out at n = 2.

AUL (Absorption Under Load) was measured through the following procedure. A weight of a pad was measured in advance, and the pad was immersed in a 0.9 wt% aqueous solution of sodium chloride for 10 minutes with a weight placed thereon to apply a load of 20 gf/m² (1.96 × 10³ Pa) on the pad. Then, the pad was pulled out with the weight placed thereon and maintained in a pulled-out state for 60 seconds to drain water therefrom. Then, a weight of the pad was measured, to thereby determine a weight of the pad increased from that before absorption as AUL (specific gravity of an aqueous solution of sodium chloride was 1). The measurement was carried out at n = 2.

**Table 1**

| | Design absorption volume (mL) | | | Absorption volume by posture (mL) | | | | |
|---|---|---|---|---|---|---|---|---|
| | Free swelling capacity | Retention capacity | AUL (20 gf/m²) | Standing | Sitting | Lying face-down | Lying on side | Lying on back |
| Product of the present invention | 320 | 200 | 120 | 300 | 280 | 250 | 200 | 150 |
| Conventional product | 800 | 500 | 250 | 250 | 220 | 180 | 140 | 140 |

Table 1 reveals that the absorbent article of the present invention (product of the present invention) absorbed a volume close to the free swelling capacity in postures of standing and sitting. Table 1 reveals that the absorbent article of the present invention absorbed a volume equal to or more than the retention capacity in postures of lying face-down and lying on side. Further, Table 1 reveals that the absorbent article of the present invention absorbed a much larger volume than that of the conventional product in any posture. Thus, the absorbent article of the present invention is excellent in body fluid absorbency compared with that of the conventional absorbent article regardless of the posture.

### <Others>

The absorbent article of the present invention may have members used in conventional absorbent articles such as an inner gather and an outer gather, in addition to the above-mentioned members.

As described above, the absorbent article and absorbent article set of the present invention are described based on the embodiments shown in the figures. However, the present invention is not limited thereto, and the structure of each of the portions may be replaced by an arbitrary structure which may exert similar functions.

### Industrial Applicability

As described above, the absorbent article of the present invention does not cause discomfort to a wearer when worn, particularly after the absorber absorbs a body fluid to be swelled, and allows disposing urine and feces separately. Thus, the absorbent article of the present invention can be suitably used as a child or adult urine-receiving article, a diaper capable of disposing urine and feces, or the like.

## Claims

1. An absorbent article, including:
a leak preventer having a sheet, and two bags provided separately on right and left of an upper side of the sheet, each of the two bags having an opening facing with each other; and
an absorber having at least one layer and containing a super absorbent polymer and capable of absorbing a body fluid, provided in each of the two bags.

2. The absorbent article according to claim 1, in which the two bags are symmetrical.

3. The absorbent article according to claim 1 or 2, further including a connecting absorber that connects the absorbers provided in each of the two bags.

4. The absorbent article according to any one of claims 1 to 3, in which the absorbers are sheet absorbers.

5. The absorbent article according to claim 4, in which the sheet absorbers contain 50 wt% or more of the super absorbent polymer.

6. The absorbent article according to claim 4 or 5, in which the sheet absorbers having multiple layers are provided in the bags.

7. The absorbent article according to any one of claims 4 to 6, in which the sheet absorbers are provided in the bags while being folded.

8. The absorbent article according to any one of claims 1 to 7, in which an area of the sheet where the two bags are not provided occupies 50% or less of an area of the entirety of the sheet in the leak preventer.

9. The absorbent article according to any one of claims 1 to 8, in which a ratio of an average left-to-right length W to an average front-to-rear length L is 0.5 or more.

10. The absorbent article according to any one of claims 1 to 9, in which an average front-to-rear length L is 20 cm or less and an area of the leak preventer seen from an upper side thereof is 400 cm² or less.

11. The absorbent article according to any one of claims 1 to 10, further including a guide sheet bridging the absorbers provided in each of the two bags.

12. The absorbent article according to claim 11, in which the guide sheet allows transfer of a body fluid between the absorbers provided in each of the two bags.

13. The absorbent article according to claim 11 or 12, in which the guide sheet extends to cover a part or entire lower surface of each of the absorbers.

14. The absorbent article according to claim 13, in which the guide sheet extends to further cover a part or entire side surface of each of the absorbers.

15. The absorbent article according to any one of claims 11 to 14, in which the guide sheet includes a concavity-and-convexity-containing sheet member having apertures forming flow paths.

16. The absorbent article according to claim 15, in which the guide sheet further includes a hydrophilic diffusion sheet laminated under or combined to a lower surface of the concavity-and-convexity-containing sheet member.

17. The absorbent article according to claim 16, in which the guide sheet further includes a body fluid impermeable sheet laminated under or combined to a lower surface of the hydrophilic diffusion sheet.

18. The absorbent article according to any one of claims 1 to 17, further including a skin contact sheet at least between the two bags of the leak preventer.

19. The absorbent article according to any one of claims 1 to 18, further including a spacer between an inner surface of an upper side of each of the bags of the leak preventer and an upper surface of each of the absorbers.

20. The absorbent article according to any one of claims 1 to 19, further including a liquid leak prevention barrier on an upper surface of the opening of each of the bags.

21. The absorbent article according to any one of claims 1 to 20, further including a fitting member for fitting the absorbent article to a body surface of a wearer between the two bags of the leak preventer.

22. The absorbent article according to claim 21, in which the fitting member is provided on an upper side of the sheet of the leak preventer.

23. The absorbent article according to claim 21 or 22, in which the fitting member is provided under a lower side of the sheet of the leak preventer.

24. The absorbent article according to any one of claims 21 to 23, in which a front-to-rear length of the fitting member is substantially the same as a front-to-rear length of the leak preventer.

25. The absorbent article according to any one of claims 21 to 23, in which a front-to-rear length of the fitting member is longer than a front-to-rear length of the leak preventer.

26. The absorbent article according to any one of claims 21 to 25, in which the fitting member is bonded to the leak preventer.

27. The absorbent article according to any one of claims 21 to 26, including the fitting member having elasticity.

28. The absorbent article according to any one of claims 21 to 27, including the fitting member having stretchability.

29. The absorbent article according to claim 28, including a plurality of the fitting members.

30. The absorbent article according to claim 28 or 29, in which the fitting member is branched on a front side and/or a rear side.

31. The absorbent article according to any one of claims 21 to 30, further including a feces-receiving portion.

32. An absorbent article set including: the absorbent article according to any one of claims 1 to 31; and a cover.

33. The absorbent article set according to claim 32, in which the cover includes a fitting member for fitting the absorbent article to a body surface of a wearer between the two bags of the leak preventer.
